# EUROPEAN PATENT APPLICATION

(11) **EP 4 628 567 A1**
(43) Date of publication of application: **08.10.2025**
(21) Application number: 23897363.0
(22) Date of filing: 01.11.2023
(51) Int. Cl.: C11B 9/00, A61K 8/34, A61L 9/01, A61Q 13/00, C07C 33/14, C11D 3/50, D06M 13/00

(54) **FRAGRANCE COMPOSITION**

(30) Priority: 29.11.2022 JP 2022190000
(71) Applicant: Takasago International Corporation, Tokyo 144-8721 (JP)
(72) Inventor: AIDA Takashi, Hiratsuka-shi, Kanagawa 254-0073 (JP)
(74) Representative: Zimmermann & Partner Patentanwälte mbB
(86) International application number: PCT/JP2023/039464
(87) International publication number: WO 2024/116707

(57) **Abstract**

The purpose of the present invention is to provide a cyclopentene ring compound having a characteristic aroma. The present invention provides: a cyclopentene ring compound that is aromatically entirely unknown, and that has a leathery/woody aroma; and a consumer product comprising the same.

## Description

### Technical Field

The present invention relates to a fragrance composition and consumer product containing a compound having a cyclopentene ring.

### Background Art

Various products with favorable scents are currently being sold as part of people's lives; however, there is a demand for a more diversified range of scented products, and there is a need to provide highly preferred fragrances. For example, compounds having a cyclopenta(te)ne ring are generally known to have a sandalwood-like aroma, and many such compounds have been developed.

### Summary of Invention

### Problems to be solved by the invention

On the other hand, since the developed compounds each have various characteristics such as fragrance, compounds with different fragrance tones are required depending on the intended use.

### Means for solution of the problems

The present inventors have discovered that a compound having a cyclopentene ring, which has not been known at all in terms of fragrance, has a leather-like and woody-like aroma, and have completed the present invention.

### Advantageous Effects of Invention

This composition can be used in a wide range for the purpose of imparting fragrance to consumer products such as fragrance products and cosmetics.

### Description of Embodiments

Hereinafter, the present invention will be described in detail.

The present invention is a fragrance composition comprising the following compound (1) as an active ingredient:

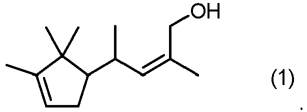

In the above compound (1), the following compound, which is a (1-R) form, is preferable:

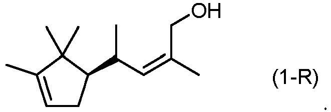

The compounds (1) and (1-R) used in the present invention can be obtained, for example, by the methods shown below, but are not limited to these methods:

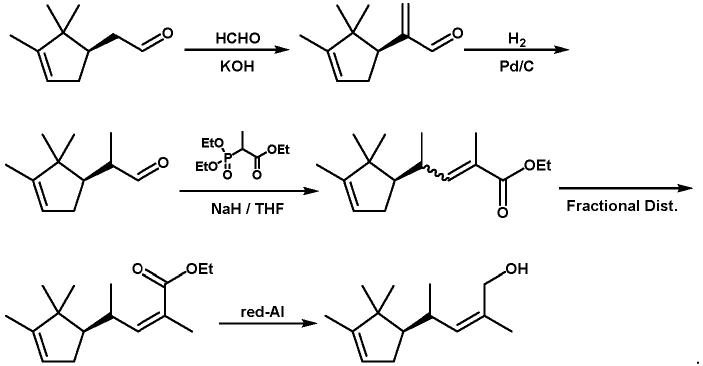

That is, formalin is reacted with campholenic aldehyde in methanol in the presence of an alkali to perform exo-methylenation, which is then hydrogenated in the presence of a palladium/carbon catalyst to obtain α-methylcampholenic aldehyde. This is converted into an α,β-unsaturated ester by a Horner-Emmons reaction using ethyl 2-(diethoxyphosphoryl)propanoate in the presence of an alkali, and only the cis form is isolated by fractional distillation or column chromatography. This ester is reduced to an alcohol with a reducing agent such as Vitride to synthesize the target compound.

The amount of the compounds (1) and (1-R) of the present invention to be blended into the fragrance composition is not particularly limited, and the blended amount usually used in this product category is preferable.

In addition, one or more other commonly used fragrance fixatives may be blended; for example, ethylene glycol, propylene glycol, dipropylene glycol, glycerin, hexylene glycol, benzyl benzoate, triethyl citrate, diethyl phthalate, Hercolyn, medium-chain fatty acid triglyceride, and the like can also be used in combination.

A commonly used formulated fragrance can be blended into the fragrance composition of the present invention. The fragrance composition thus obtained can provide a fresh and highly palatable fragrance. In addition, the fragrance composition of the present invention can be blended as a fragrance component in, for example, fragrance products, cosmetics, basic cosmetics, finishing cosmetics, hair cosmetics, sunscreen cosmetics, medicated cosmetics, hair care products, soaps, body washes, bath agents, clothing detergents, clothing softeners, cleaning agents, kitchen detergents, bleaches, air care products, repellents, and miscellaneous goods in the amounts usually blended in this industry to impart its unique fragrance and enhance the commercial value of the product.

For example, fragrance products include perfumes, eau de parfums, eau de toilettes, eau de colognes, etc.; basic cosmetics include facial cleansing creams, vanishing creams, cleansing creams, cold creams, massage creams, milky lotions, lotions, beauty serums, packs, makeup removers, etc.; finishing cosmetics include foundations, lipsticks, lip creams, etc.; hair cosmetics include hair tonics, hair liquids, hair sprays, etc.; sunscreen cosmetics include suntan products, sunscreen products, etc.; medicated cosmetics include antiperspirants, body deodorants, after-shaving lotions and gels, permanent wave agents, medicated soaps, medicated shampoos, medicated skin cosmetics, etc.

Hair care products include shampoos, rinses, 2-in-1 shampoos, conditioners, treatments, hair packs, etc.; soaps include cosmetic soaps, bath soaps, etc.; body washes include body soaps, body shampoos, hand soaps, etc.; bath agents include bath additives (such as bath salts, bath tablets, and bath liquids), foam baths (such as bubble baths), bath oils (such as bath perfumes and bath capsules), milk baths, bath jellies, bath cubes, etc.

Detergents include heavy-duty detergents for clothing, light-duty detergents for clothing, liquid detergents, laundry soaps, compact detergents, powder soaps, etc.; softeners include liquid softeners, dryer sheets, etc.; cleaning agents include cleansers, furniture care, house cleaners, toilet cleaners, bathroom cleaners, glass cleaners, mold removers, drain cleaners, etc.; kitchen detergents include kitchen soaps, synthetic kitchen soaps, dishwashing detergents, dishwasher detergents, etc.; bleaches include oxidizing bleaches (such as chlorine-based bleaches and oxygen-based bleaches), reducing bleaches (such as sulfur-based bleaches), optical bleaches, etc.; air care products include aerosols (spray type, powder spray), candles, wax melts, car fresheners, stationary, spray type, and plug-in type deodorizers/air fresheners (such as solid type, gel type, and liquid type); pet care products include pet deodorizers, pet toilet deodorizers, pet shampoos, and pet conditioners; and miscellaneous goods include various forms such as tissue paper, toilet paper, disinfectant wipes, and disinfectant sprays.

When the fragrance composition of the present invention is used in the above-mentioned products, it may be used as is, or in a liquid state dissolved in, for example, alcohols or polyhydric alcohols such as propylene glycol or glycerin; in a solubilized or dispersed state using surfactants, such as nonionic surfactants, anionic surfactants, cationic surfactants, and amphoteric surfactants; or in a microencapsulated state obtained by treatment with an encapsulating agent, etc.; any shape can be selected and used depending on the purpose.

Furthermore, the above fragrance composition may be stabilized and made sustained-release for use by being included in an inclusion agent such as cyclodextrin. These are suitably selected and used according to the form of the final product, for example, liquid, solid, powder, gel, mist, aerosol, etc.

### Examples

The present invention will be specifically described below with reference to Examples, etc., but the present invention is not limited thereto. The following devices were used to measure physical properties in the Examples.
NMR: DRX500 (manufactured by Bruker)
GC/MS: HP5977A (manufactured by Agilent Technologies)
Column: Inertcap-1 (length 30 m x inner diameter 0.25 mm, film thickness 0.25 µm) (manufactured by GL Sciences Inc.)

### (Example 1)

### Synthesis of (R, Z)-2-methyl-4-(2,2,3-trimethylcyclopent-3-en-1-yl)pent-2-en-1-ol (1) Synthesis of (S)-2-(2,2,3-trimethylcyclopent-3-en-1-yl)acrylaldehyde

A solution of 0.79 g (19.7 mmol) of sodium hydroxide dissolved in 150 ml of methanol was added to 30 g (197.1 mmol) of campholenaldehyde synthesized according to a known method using (1S,5S)-α-pinene with an optical purity of 97% e.e. as a raw material, while maintaining the temperature at 10°C. 16.79 g (206.9 mmol) of formalin (37% aqueous solution) was added thereto. The solution was heated in an oil bath at 50°C and reacted for 2 hours, at which point the raw material disappeared.

After quenching by adding acetic acid, methanol was distilled off under reduced pressure using a rotary evaporator and extracted with toluene. After washing with water three times, vacuum distillation was performed using a Claisen-type distiller.

Distillation was carried out at an oil bath temperature of 100°C and a reduced pressure of 1.1 x 10³ Pa at 73-75°C to obtain 26.8 g of a colorless oil with a purity of 97.6% of (S)-2-(2,2,3-trimethylcyclopent-3-en-1-yl)acrylaldehyde.
1H NMR (500 MHz, CDCl3, δ) ppm: 0.68 (s, 3H), 1.04 (s, 3H), 1.59-1.63 (m, 3H), 2.31-2.38 (m, 2H), 3.20 (dt, J=0.8 Hz, J=8.0 Hz, 2H), 5.30 (d, J=1.6 Hz, 1H), 6.12 (d, J=0.8 Hz, 1H), 6.35 (s, 1H), 9.58 (s, 1H).
13C NMR (125 MHz, CDCl3, δ) ppm: 12.76 (q), 21.63 (q), 26.45 (q), 34.62 (t), 46.40 (d), 48.12 (s), 121.38 (d), 135.59 (t), 147.29 (s), 151.27 (s), 195.28 (d).
MS (m/e): 141, 149, 136, 131, 121, 107, 95, 93, 91, 79, 77, 67, 65, 55, 53.

### (2) Synthesis of (S)-2-((R)-2,2,3-trimethylcyclopent-3-en-1-yl)propanal

100 mg of a palladium-carbon (5% loading), 79.5 ml of ethyl acetate, and 26.5 g (161.3 mmol) of (S)-2-(2,2,3-trimethylcyclopent-3-en-1-yl)acrylaldehyde obtained in (1) above were placed in a 200 ml autoclave, and hydrogenation was carried out at a hydrogen pressure of 2.5 MPa and 60°C until hydrogen consumption ceased.

After filtering the reaction solution through Celite, the solvent was distilled off using a rotary evaporator, and then vacuum distillation was performed using a Claisen-type distiller.

Distillation was carried out at an oil bath temperature of 100°C and a reduced pressure of 1.0 x 10³ Pa at 77-82°C to obtain 24.8 g of a colorless oil with a purity of 98.2% of (S)-2-((R)-2,2,3-trimethylcyclopent-3-en-1-yl)propanal.
1H NMR (500 MHz, CDCl3, δ) ppm: 0.93 (s, 3H), 1.11 (s, 3H), 1.17 (d, J=6.8 Hz, 3H), 1.57-1.63 (m, 3H) 1.92-2.10 (m, 2H), 2.27-2.37 (m, 1H), 2.50-2.59 (m, 1H), 5.19-5.28 (m, 1H), 9.63 (d, J=3.6 Hz, 1H).
13C NMR (125 MHz, CDCl3, δ) ppm: 12.39 (q), 13.90 (q), 19.84 (q), 27.10 (q), 33.44 (t), 47.55 (s), 47.95 (d), 51.70 (d), 121.39 (d), 148.34 (s), 205.12 (d).

The mass spectrum is separated into two diastereomers.
MS (m/e): 166, 151, 133, 123, 108, 93, 81, 79, 77, 67, 55, 41. (minor)
MS (m/e): 166, 151, 133, 123, 108, 93, 81, 79, 77, 67, 55, 41. (major)

### (3) Synthesis of ethyl (R)-2-methyl-4-(2,2,3-trimethylcyclopent-3-en-1-yl)pent-2-enoate (cis/trans mixture)

6.16 g (154.1 mmol) of sodium hydride (60% oil dispersion) was dispersed in 244 ml of anhydrous tetrahydrofuran, and 36.71 g (154.1 mmol) of ethyl 2-(diethoxyphosphoryl)propionate was added dropwise thereto over 1 hour at room temperature. After the dropwise addition, the mixture was heated in an oil bath at 60°C, and 24.4 g (146.8 mmol) of (S)-2-(2,2,3-trimethylcyclopent-3-en-1-yl)propanal obtained in the previous section was added dropwise over 1 hour. The raw material disappeared 1 hour after the dropwise addition.

After distilling off the solvent using a rotary evaporator, the reaction was quenched by adding water, extracted with toluene, washed twice with water, and the solvent was distilled off using a rotary evaporator to obtain a crude product of ethyl (R)-2-methyl-4-(2,2,3-trimethylcyclopent-3-en-1-yl)pent-2-enoate (cis:trans=1:1) as a pale yellow oil.

### (4) Synthesis of ethyl (R,Z)-2-methyl-4-(2,2,3-trimethylcyclopent-3-en-1-yl)pent-2-enoate

The crude product of ethyl (R)-2-methyl-4-(2,2,3-trimethylcyclopent-3-en-1-yl)pent-2-enoate obtained in (3) above was subjected to silica gel column chromatography (solvent: hexane: ethyl acetate=9:1) to obtain 19.91 g (79.5 mmol) of ethyl (R,Z)-2-methyl-4-(2,2,3-trimethylcyclopent-3-en-1-yl)pent-2-enoate as a colorless oil.

Note that the cis form has a lower polarity than the trans form and comes out first in column chromatography.
1H NMR (500 MHz, CDCl3, δ) ppm: 0.73 (s, 3H), 0.98 (d, J=6.7 Hz, 3H), 1.29 (t, J=7.1 Hz, 3H), 1.53-1.59 (m, 6H), 1.60-1.69 (m, 1H), 1.85-1.90 (m, 1H), 1.91 (d, J=1.5 Hz, 3H), 2.21-2.29 (m, 3H), 3.22-3.33 (m, 1H), 4.15-4.26 (m, 2H), 5.20-5.24 (m1H), 5.77-5.83 (m, 1H).
13C NMR (125 MHz, CDCl3, δ) ppm: 12.56 (q), 14.29 (q), 19.37 (q), 19.86 (q), 20.69 (q), 26.41 (q), 34.63 (d), 35.35 (t), 56.63 (d), 60.02 (t), 121.11 (d), 124.39 (s), 149.04 (s), 149.35 (d), 168.33 (s).

The mass spectrum is separated into two diastereomers.
MS (m/e): 250, 189, 180, 177, 161, 142, 135, 121, 114, 112, 109, 95, 81, 67, 43, 41. (major)
MS (m/e): 250, 235, 207, 189, 180, 161, 142, 135, 121, 114, 113, 109, 95, 81, 79, 67, 55, 43, 41. (minor)

### (5) Synthesis of (R,Z)-2-methyl-4-(2,2,3-trimethylcyclopent-3-en-1-yl)pent-2-en-1-ol

19.7 g (78.6 mmol) of ethyl (R,Z)-2-methyl-4-(2,2,3-trimethylcyclopent-3-en-1-yl)pent-2-enoate obtained in (4) above was dissolved in 80 ml of toluene, and 15.9 g (51.1 mmol) of a 65% toluene solution of sodium bis(2-methoxyethoxy)aluminum hydride was added dropwise thereto over 1 hour. After stirring for 1 hour after the dropwise addition, the reaction was quenched in hydrochloric acid, extracted with toluene, and then washed three times with water.

The solvent was distilled off using a rotary evaporator and purified through a short silica gel column to obtain 14.58 g (70.0 mmol) of the target (R,Z)-2-methyl-4-(2,2,3-trimethylcyclopent-3-en-1-yl)pent-2-en-1-ol as a colorless oil.
1H NMR (500 MHz, CDCl3, δ) ppm: 0.75 (s, 3H), 0.92 (d, J=6.7 Hz, 3H), 0.99 (s, 3H), 1.53-1.60 (m, 4H), 1.61-1.69 (m, 1H), 1.81 (d, J=1.5 Hz, 3H), 1.83-1.93 (m, 1H), 2.21-2.29 (m, 1H), 2.47-2.57 (m, 1H), 4.13-4.25 (m, 2H), 5.19-5.26 (m, 1H).
13C NMR (125 MHz, CDCl3, δ) ppm: 12.53 (q), 19.22 (q), 21.08 (q), 21.49 (q), 26.51 (q), 34.05 (d), 34.92 (t), 46.92 (s), 56.14 (d), 62.06 (t), 121.14 (d), 130.57 (s), 135.96 (d), 149.06 (s).
MS (m/e): 208, 193, 175, 135, 121, 109, 108, 95, 93, 81, 79, 67, 55, 43, 41.

### (Example 2: Fragrance Composition)

A fragrance composition was prepared according to the formulation in Table 1 below using the compound synthesized in Example 1 above.

**Table 1**

| Formulation | (Parts by Weight) |
|---|---|
| Allyl Caproate | 14 |
| L-Citronellyl Nitrile | 6 |
| CYCLAPROP | 30 |
| α-Damascone | 12 |
| Ethyl 2-Methylbutyrate | 10 |
| Ethyl Methylphenylglycidate | 8 |
| Eugenol | 2 |
| FRUITATE | 10 |
| Geranyl Acetate | 16 |
| HEDIONE | 100 |
| HELIOBOUQUET | 6 |
| cis-3-Hexenol | 2 |
| Hexyl Acetate | 30 |
| Hexyl Cinnamic Aldehyde | 70 |
| Hexyl Salicylate | 50 |
| HINDINOL | 8 |
| cis-Jasmone | 1 |
| Linalyl Acetate | 10 |
| Orange Oil | 50 |
| L-ORANTHA SUPER 1.0% dipropylene glycol solution | 6 |
| ORBITONE | 20 |
| PEONILE | 14 |
| PHENOXANOL | 20 |
| Raspberry Ketone | 36 |
| ROSYRANE SUPER 1.0% dipropylene glycol solution | 4 |
| Tetrahydrolinalool | 100 |
| TRIPLAL | 4 |
| γ-Undecalactone | 20 |
| VERDOX | 60 |
| Yara Yara | 2 |
| Dipropylene Glycol | 209 |
| Compound of Example 1 | 70 |
| Total | 1000 |

### (Example 3: Liquid Detergent)

A liquid detergent (100 g) scented with 0.5% of the fragrance composition of Example 2 was prepared according to the formulation in Table 2 below.

**Table 2**

| Formulation (Ingredients) | (Amount Blended (g)) |
|---|---|
| Polyoxyalkylene Alkyl Ether | 50.00 |
| Water | 35.13 |
| Butyl CARBITOL | 8.00 |
| Linear Alkylbenzene Sulfonate | 2.50 |
| p-Toluenesulfonic Acid | 1.00 |
| Polyethylene Glycol | 1.00 |
| Monoethanolamine | 1.00 |
| Coconut Oil Fatty Acid | 0.10 |
| Citric Acid | 0.22 |
| Sodium Benzoate | 0.50 |
| BHT | 0.05 |
| Fragrance Composition of Example 2 | 0.50 |
| Total | 100.00 |

### (Example 4: Shampoo)

A shampoo (100 g) scented with 1.0% of the fragrance composition of Example 2 was prepared according to the formulation in Table 3 below.

**Table 3**

| Formulation (Ingredients) | (Amount Blended (g)) |
|---|---|
| Sodium Polyoxyethylene Lauryl Ether Sulfate | 14.00 |
| Lauramidopropyl Betaine | 4.00 |
| Coconut Oil Fatty Acid Diethanolamide | 3.00 |
| Cationic Cellulose | 0.50 |
| Ethylene Glycol Distearate | 1.00 |
| Ethyl Parahydroxybenzoate | 0.25 |
| Citric Acid | Appropriate Amount |
| Fragrance Composition of Example 2 | 1.00 |
| Purified Water | Balance |
| Total | 100.00 |

### (Example 5: Body Shampoo)

A body shampoo (100 g) scented with 0.95% of the fragrance composition of Example 2 was prepared according to the formulation in Table 4 below.

**Table 4**

| Formulation (Ingredients) | (Amount Blended (g)) |
|---|---|
| Triethanolamine | 9.00 |
| Lauric Acid | 6.00 |
| Myristic Acid | 9.00 |
| Disodium Laureth Sulfosuccinate (1E.0.) (42%) | 10.00 |
| Alkyl (C8-16) Glucoside | 8.00 |
| Glyceryl Laurate | 1.00 |
| 2-Hydroxyethyl Distearate | 2.50 |
| Coconut Oil Fatty Acid Diethanolamide | 3.00 |
| Propylene Glycol | 5.00 |
| Dibutylhydroxytoluene | 0.05 |
| Disodium Edetate | 0.10 |
| Ethyl Parahydroxybenzoate | 0.20 |
| Methyl Parahydroxybenzoate | 0.10 |
| Fragrance Composition of Example 2 | 0.95 |
| Purified Water | Balance |
| Total | 100.00 |

## Claims

1. A fragrance composition comprising a compound represented by the following formula (1):

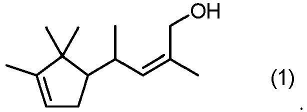

2. The fragrance composition according to claim 1, comprising a compound represented by the following formula (1-R): 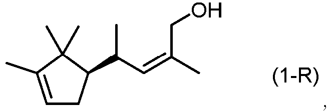 wherein an enantiomeric excess of an R-form is in a range of 1% e.e. to 90% e.e.

3. A product scented with the fragrance composition according to claim 1 or 2.

4. The scented product according to claim 3, wherein the product is at least one selected from fragrance products, cosmetics, basic cosmetics, finishing cosmetics, hair cosmetics, sunscreen cosmetics, medicated cosmetics, hair care products, soaps, body washes, bath agents, clothing detergents, clothing softeners, cleaning agents, kitchen detergents, bleaches, aerosols, deodorizers/air fresheners, repellents, and miscellaneous goods.

5. A compound represented by the following formula (1-R):
